(19) Europäisches Patentamt / European Patent Office / Office européen des brevets

(11) **EP 3 591 377 B1**

(12) **EUROPEAN PATENT SPECIFICATION**

(45) Date of publication and mention of the grant of the patent:
**16.06.2021 Bulletin 2021/24**

(51) Int Cl.:
**G01N 15/08** (2006.01)   **G01N 33/483** (2006.01)
**G01N 33/50** (2006.01)

(21) Application number: **18181779.2**

(22) Date of filing: **04.07.2018**

(54) **ELECTROCHEMICAL DETERMINATION OF THE PERMEABILITY OF BIOLOGICAL MEMBRANES AND CELLULAR LAYERS**

ELEKTROCHEMISCHE BESTIMMUNG DER PERMEABILITÄT VON BIOLOGISCHEN MEMBRANEN UND ZELLSCHICHTEN

DÉTERMINATION ÉLECTROCHIMIQUE DE LA PERMÉABILITÉ DE MEMBRANES BIOLOGIQUES ET DE COUCHES CELLULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**08.01.2020 Bulletin 2020/02**

(73) Proprietors:
• **Universität Regensburg**
  **93053 Regensburg (DE)**
• **nanoAnalytics GmbH**
  **48149 Münster (DE)**

(72) Inventors:
• **WEGENER, Joachim**
  **93051 Regensburg (DE)**
• **URBAN, Florian**
  **93051 Regensburg (DE)**
• **HAJEK, Kathrin**
  **93049 Regensburg (DE)**
• **ANCZYKOWSKI, Boris**
  **48147 Münster (DE)**

(74) Representative: **Lucke, Andreas**
  **Boehmert & Boehmert**
  **Anwaltspartnerschaft mbB**
  **Pettenkoferstrasse 22**
  **80336 München (DE)**

(56) References cited:
DE-A1-102007 003 585

• HAJEK KATHRIN ET AL: "Independent impedimetric analysis of two cell populations co-cultured on opposite sides of a porous support", EXPERIMENTAL CELL RESEARCH, ELSEVIER, AMSTERDAM, NL, vol. 351, no. 1, 10 January 2017 (2017-01-10), pages 121-126, XP029912795, ISSN: 0014-4827, DOI: 10.1016/J.YEXCR.2017.01.003
• WEGENER J ET AL: "Barrier function of porcine choroid plexus epithelial cells is modulated by cAMP-dependent pathways in vitro", BRAIN RESEARCH, ELSEVIER, AMSTERDAM, NL, vol. 853, 17 January 2000 (2000-01-17), pages 115-124, XP002393349, ISSN: 0006-8993, DOI: 10.1016/S0006-8993(99)02317-3
• WEGENER J ET AL: "IMPEDANCE ANALYSIS OF EPITHELIAL AND ENDOTHELIAL CELL MONOLAYERS CULTURED ON GOLD SURFACES", JOURNAL OF BIOCHEMICAL AND BIOPHYSICAL METHODS, AMSTERDAM, NL, vol. 32, no. 3, 10 July 1996 (1996-07-10), pages 151-170, XP001183692, ISSN: 0165-022X, DOI: 10.1016/0165-022X(96)00005-X
• JOHN L WOOD ET AL: "REFINEMENTS IN THE SHORT-CIRCUIT TECHNIQUE AND ITS APPLICATION TO ACTIVE POTASSIUM TRANSPORT ACROSS THE CECROPIA MIDGUT", J. EXP. BIOL, vol. 77, 1 January 1978 (1978-01-01), pages 123-140, XP055707575,

## Description

FIELD OF THE INVENTION

The invention relates to a method and a device to determine the permeability of a sample in diffusive media.

BACKGROUND

Determining the permeability of a sample is of great importance in biology and chemistry, for example for biomedical applications to study the delivery of pharmacological substances.

For the development of novel drugs, one of the main challenges is the efficient delivery of the drug within the human body, i.e. to facilitate the transfer of the pharmacologically active substance to the desired location, for example a particular organ, after administration of the drug. For this, the substance has to permeate multiple biological barriers, e.g. epithelial or endothelial tissue, which can limit the overall effectiveness of the drug.

For this reason, knowledge about the ability of a given substance to permeate biological membranes is indispensable for drug development, and extensive studies have to be performed to quantify the permeability of the relevant types of membranes. Experimental methods and tools for performing *ex vivo* measurements of the transport properties of isolated cell layers under controlled laboratory conditions are of great relevance for this.

To determine the permeability of a membrane or cell layer, two main techniques are used: (a) measuring the electrical resistance of the membrane or cell layer in an electrolyte solution and (b) examining the diffusion of molecular probes through the membrane or cell layer, see D. Günze/ et al., Curr. Top. Membr. 65, 39-78 (2010); and J. Wegener et al., Cell Tissue Res. 335, 485-514 (2014).

The electrical resistance can be measured by placing the membrane or cell layer in a solution containing an electrolyte and applying a transverse voltage across the membrane or cell layer, e.g. using two electrodes. The voltage induces a motion of the ions in the solution, which have to pass through the membrane or cell layer to get from one electrode to the other. The electrical resistance between the electrodes is thus a measure for the ion permeability of the membrane or cell layer.

The diffusion of molecular probes through the membrane or cell layer can be studied by placing the membrane between two solutions containing different concentrations of the molecular probes. Due to the concentration gradient, diffusion between the two solutions will cause a net current of molecules, which over time leads to an equilibration of the concentration throughout the system. The duration of this equilibration depends on the permeability of the membrane or cell layer for that particular molecular probe, which can for example be quantified by the molecular permeability coefficient $P_E$.

To measure the concentration of molecular probes during the diffusion process, various techniques have been developed, e.g. labeling of the molecular probes with fluorophores, see S. Sanders et al., Epithelial Cell Biol. 4, 25-34 (1995) or radioactive substances as described in H. Ghandehari et al., J. Pharm. Exp. Ther. 280, 747-753 (1997).

These techniques, however, require complicated experimental setups and handling and offer only a very limited time resolution. They are thus not suitable for a fast and cost-efficient analysis of samples in large quantities.

J. Wegener et al., J. Biochem. Biophys. Methods 32,151 (1996) describes a method and setup for the impedance analysis of cell monolayers on gold surfaces. A plurality of gold electrodes is deposited on a microscopy slide, on which cell monolayers are cultured. Using a counterelectrode, the transepithelial and transendothelial electrical resistances (TEER) of the cell layers can be determined in a spatially resolved way.

K. Hajek and J. Wegener, Experimental Cell Research 351, 121 (2017) discloses a method and apparatus for measuring the transepithelial or -endothelial electrical resistance of two cell populations grown on opposite sides of a permeable filter support. To determine the TEER of each cell population individually, one side of the filter is coated with a gold electrode, which is covered by one of the cell populations.

J. Wegener et al., Brain Research 853, 115 (2000) reports on studies of the barrier function of cell layers, wherein the permeation rate of probe molecules is determined by fluorescence measurements in addition to an electrical measurement of the TEER.

J. L. Wood et al., J. Exp. Biol. 77, 123-140 (1978) *discloses a method for short-circuit current measurements on a sample of epithelial tissue, wherein a voltage across the sample* is *measured using two electrodes on opposite sides of the sample and corrected using a third electrode arranged on one of the sides.*

SUMMARY OF THE INVENTION

The object of the invention is thus to provide a method and a device for determining the permeability of a sample in a fast and simple manner which would be more suitable for automatization.

This aim is achieved by disclosing a method and a device according to Claim 1 and 10, respectively. Embodiments of the present invention are detailed in the dependent claims.

The method for determining the permeability of a sample through observation of a diffusive process by electrochemical means J comprises the following steps: (1) placing the sample between a first and a second volume to form a barrier between the two volumes; (2) providing a first medium in the first volume and a second medium in the second volume, wherein at least one of the first and second media contains at least one kind of electrochemically active molecules, and wherein the concentrations of said electrochemically active molecules in said first and second media are different from each other; (3) measuring a first electrical signal between at least two electrodes in contact with the second volume at different points in time while the electrochemically active molecules are allowed to diffuse through the sample, said first electrical signal depending on the concentration of electrochemically active molecules at at least one position in the second volume and (4) determining from said measured first electrical signal at the different points in time a first measure for the permeability of the sample, said first measure characterizing, a permeability of the sample with respect to said electrochemically active molecules. The numbering of the steps above is for clarity only and does not indicate a certain order. As far as technically feasible, the steps can be permuted and the method and any embodiment thereof can be performed in an arbitrary order.

This method provides a way to quantify the permeability of a sample through the observation of a diffusive process based on measurements of the concentration of probe molecules by electrochemical means. This allows for fast and accurate measurements with high temporal resolution. Additionally, it facilitates the combination with existing techniques for measuring the electrical resistance in a single experimental setup, thereby avoiding a transfer of the sample between the two setups or the use of separate samples. The method enables an in situ determination of the permeability without requiring to take samples of the media during the measurement as in K, Morimoto et al., Pharm. Res. 10, 1668-1674 (1993), and thus minimizes interference with the measurement, e.g. through temperature fluctuations. The process can be fully automated, making it suitable for a fast and cheap analysis of large numbers of samples. Furthermore, the method can be used for a local probing of the sample's permeability for the molecular probe.

In the first step, the sample is placed between a first volume and a second volume to separate the two volumes, i.e. the sample forms the main diffusive barrier between the volumes, which limits the exchange of probe molecules. The sample can completely separate the two volumes by covering the entire boundary surface between the volumes, or it can partially separate them, provided that particle exchange is dominated by the samples' diffusive properties.

The first and second media contain at least one kind of electrochemically active molecules, the probe molecules, in different concentrations. The concentration of probe molecules can be zero in one of the media and nonzero in the other medium. Diffusion of probe molecules through the sample leads to an equilibration between the media until the concentration in the first and second media become equal. The equilibration rate depends on the sample's permeability with respect to the probe molecules. Here, the term electrochemically active molecules covers all substances that can give rise to an electrical signal between two or more electrodes in contact with a medium containing the substance, wherein the electrical signal can e.g. be a voltage or a current. These substances include, but are not limited to, molecules or ions that can undergo oxidation processes and/or reduction processes, for example ferrocenemethanol $C_{11}H_{12}FeO$, ferrocyanide $[Fe(CN)_6]^{4-}$, ferricyanide $[Fe(CN)_6]^{3-}$ or mixtures thereof.

The media can be any media in which the probe molecules can move freely and with a larger diffusion coefficient than in the sample under test. In one example, at least one of the media can be a fluid, in particular a liquid, or a gel.

According to the present invention, a first electrical signal is measured between at least two electrodes in contact with the second volume at at least two different points in time during the equilibration process after the media are provided in the volumes. This first electrical signal depends on the concentration of probe molecules at at least one position in the second volume such that a measure for the concentration can be obtained from the electrical signal. The signal can depend on the concentration in the vicinity of a surface of one or more of the at least two electrodes or it can be determined by the average concentration in the second medium.

The first electrical signal can for example be a current between two electrodes in response to a voltage applied between the electrodes. In another example, the first electrical signal can be a voltage between the at least two electrodes, which arises due to the presence of probe molecules, e.g. by an electrochemical reaction at a surface of an electrode. This voltage may be measured without a current flow between the electrodes, e.g. with a potentiometer. Alternatively, a current can be forced through the sample and the resulting voltage between the electrodes can be measured as the first electrical signal.

The first electrical signal can be measured at at least two different points in time following the insertion of the media into the two volumes. The first electrical signal can in particular be recorded as a continuous or quasi-continuous time trace during the diffusion process.

A first measure characterizing at least in part the permeability of the sample with respect to the probe molecules is obtained from the first electrical signal. This measure can for example be a rate of change of the first electrical signal, i.e. the difference in the first electrical signal between at least two different points in time, or a rate of change of a quantity derived from the first electrical signal, e.g. an electrical impedance. In another example, the determination of the first measure can involve modelling of the diffusion process, e.g. fitting a fit function to the first electrical signal or quantities extracted from the first electrical signal like an impedance and obtaining the first measure from the fit parameters. This modelling can also take into account additional effects like the initial concentration of probe molecules in the first and second media or contributions to the measured signal arising from the impedance of the second medium or the capacitance of the electrodes. The first measure can be a relative quantity in arbitrary units for comparison of different samples in similar experimental setups, or it can be an absolute quantity like the molecular permeability coefficient or the diffusion constant of the probe molecules within the sample.

In one example of the present invention, the method additionally comprises measuring a second electrical signal between at least one electrode in contact with the first volume and at least one electrode in contact with the second volume, wherein at least one of the first and second media is an electrolyte solution. Said second electrical signal depends on the electrical conductivity of the sample with respect to an electrolyte current. From said second electrical signal a second measure for the permeability of the sample is determined, which characterizes, at least in part, a permeability of the sample with respect to ions of said electrolyte.

The measurement of the second electrical signal can be conducted before or after the measurement of the first electrical signal so as to not influence the diffusion process e.g. by a voltage across the sample, which can be applied in order to measure the second electrical signal. Furthermore, the second electrical signal can be measured when the first and second media are in or at least close to equilibrium and in particular contain the same concentration of ions to avoid spurious effects due to a simultaneous diffusion driven by a concentration gradient. In another example, the first and second electrical signal can be measured alternatingly over a period of time. If the measurement of the second electrical signal involves the application of a voltage, the influence of this voltage on the diffusion of the electrochemically active molecules can be reduced e.g. by using a short measurement duration, an AC voltage with a frequency that is fast compared to the time scale of the diffusion or a voltage with a small amplitude. Alternatively, the first and second signal can be measured simultaneously if their individual contributions can be separated technically, e.g. by using different AC frequencies for the two measurements or by recording full impedance spectra with subsequent data modelling and thereby separation of the different contributions.

The ions of said electrolyte can be the electrochemically active molecules or can be different from the electrochemically active molecules. In particular, the ions can be smaller than the electrochemically active molecules and the concentration of ions in the first and second media can be the same. The ions can be inorganic ions. The ions of the electrolyte and the electrochemically active molecules are chosen such that the determination of the first and second measure for the permeability of the sample is possible in the presence of both kinds of substances. If the ions do not correspond to the electrochemically active molecules, their presence might influence the first electrical signal. However, this contribution can be separated from the contribution of the electrochemically active molecules e.g. if the concentration of ions in the second medium is constant over time and only manifests in a constant background signal or if the permeability of the sample is different with respect to the ions and the electrochemically active molecules, leading to different equilibration time scales. The ions and their concentrations in the media can further be chosen such that the presence of the electrochemically active molecules and the gradient in their concentration has a negligible effect on the second electrical signal, e.g. if the diffusion of the ions through the sample is much faster than for the electrochemically active molecules or if the concentration of ions is much higher than the one of the electrochemically active molecules.

The second electrical signal can for example be a current between one electrode in contact with the first volume and one electrode in contact with the second volume in response to a voltage applied between the electrodes. Alternatively, the second electrical signal can be a voltage between these electrodes that is required to induce a certain current through the sample.

A second measure, which characterizes at least in part the permeability of the sample with respect to the ions of the electrolyte, is extracted from the second electrical signal. This measure can for example be the electrical impedance of the sample, in particular the electrical resistance, which quantifies the sample's electrical resistance with respect to a transverse electrolyte current through the sample. The electrical resistance can be determined from the DC electrical resistance between the electrodes by comparison with a measurement without the sample. In another example, the electrical resistance is determined from an impedance spectrum, which is obtained from a measurement of the current in response to an AC voltage as a function of the frequency or from a corresponding measurement of the voltage in response to an AC current that is forced through the sample. This determination can involve a modelling of the electrochemical components between the electrodes by an electrochemical model, which can account for e.g. the capacitance of the sample as well as the impedance of the media. In another example the electrical resistance is determined from AC impedance measurements at a single, fixed frequency or at a few, predefined frequencies. This approach typically requires measuring a reference, i.e. a sample-free configuration, for comparison.

The electrochemically active molecules might contribute to the second electrical signal. This can be avoided by using a first and second media which do initially not contain the electrochemically active molecules and adding the electrochemically active molecules to at least one of the media after the measurement of the second electrical signal and before the measurement of the first electrical signal.

In one example of the present invention, the sample can consist, at least in part, of biological cells. The sample can be a membrane containing a single layer or multiple layers of cells. In particular, the sample can comprise epithelial or endothelial cells and can be designed to mimic the function of epithelial or endothelial tissue in the human body.

As mentioned above, the first measure may be a permeability coefficient of the sample and the second measure maybe an electrical resistance of the sample.

The method according to an embodiment of the present invention can further comprise measuring a third electrical signal between at least two electrodes in contact with the second volume at different points in time during the diffusion of the electrochemically active molecules through the sample, wherein at least one of said at least two electrodes is different from the at least two electrodes used for the measurement of said first electrical signal.

The third electrical signal depends on the concentration of electrochemically active molecules at at least one position in the second volume. The signal can depend on the concentration in the vicinity of a surface of one or more of the at least two electrodes used for the measurement of the third electrical signal or it can be determined by the average concentration in the second medium.

The third electrical signal can be of the same type as the first electrical signal, e.g. an AC or DC current or voltage. The measurement of the third electrical signal can be performed simultaneously with the measurement of the first electrical signal. Alternatively, the two measurements can be conducted in an alternating fashion to avoid mutual crosstalk.

From said third electrical signal, a third measure for the permeability of the sample with respect to the electrochemically active molecules can be obtained, which characterizes at least in part a permeability of the sample with respect to said electrochemically active molecules. This measure can be of the same type as the first measure and can be obtained in the same way.

This method can be extended accordingly to include a measurement of at least one more additional electrical signal between at least two electrodes in contact with the second volume at different points in time while the electrochemically active molecules are allowed to diffuse through the sample, wherein at least one of the said at least two electrodes is different from the at least two electrodes used for the measurement for any of the other electrical signals.

In one example of the present invention, the first and third electrical signal can be used to obtain information pertaining the spatial distribution of a permeability of the sample with respect to the electrochemically active molecules. The first and third electrical signal can predominantly depend on the permeability of different parts of the sample, e.g. if the corresponding electrodes are located in different parts of the second volume. In particular when a plurality of such signals is measured employing a plurality of electrodes, detailed information about the distribution of the permeability throughout the sample can be obtained.

From the information on the spatial distribution, a measure for the homogeneity of the sample can be determined, e.g. the standard deviation of the corresponding permeability measures. This measure can be compared to predefined criteria characterizing the sample's homogeneity. This enables the identification of potentially defective samples, which might for example contain holes or impurities. In particular, it allows for an assessment of the reliability of the second measure for the sample's permeability, which is otherwise difficult to obtain if the respective measurements are conducted in different setups and using different samples. Based on the comparison, a decision can be made as to whether or not to repeat the measurements with a new sample.

As mentioned above, at least one of the two media may be a fluid, in particular a liquid, or a gel.

Moreover, the electrochemically active molecules and/or the ions of the electrolyte may be chosen such that a transfer through the sample involves paracellular transport. Preferably, paracellular transport constitutes the dominant way of transport through the sample, e.g. through the use of molecules with a high water solubility to prevent a transcellular transport through the cellular lipid bilayer.

In a preferred embodiment, said electrochemically active molecules are larger than the ions of said electrolyte.

In a preferred embodiment, at least one of said electrical signals is a current between at least two of said electrodes in response to a voltage applied between the electrodes. The applied voltage can be a DC voltage such that the current depends on the DC electrical resistance between the electrodes. Alternatively, the voltage can be an AC voltage, giving rise to an AC current depending on the impedance between the electrodes. The frequency of the AC voltage may be varied during the measurement to determine the impedance as a function of the applied frequency. The applied voltage may be chosen such that the sample is not damaged during the measurement and in particular such that the diffusion process and the permeability of the sample are not affected by the application of the voltage.

In one example of the present invention, a measure for an electrical impedance can be determined from at least one of the first and second electric signals. This measure can for example be a magnitude or phase of the electrical impedance or a combination thereof. The electrical impedance may be an impedance between the at least two electrodes used for the measurement of the respective first or second electric signal or may be an impedance of the sample obtained from the impedance between the respective electrodes. The measure for the electrical impedance may be determined at a single, fixed frequency of an AC voltage or an AC current that is applied between the respective electrodes or at a few, predefined frequencies. In particular, the measure of the electrical impedance may be determined as a function of a frequency of an AC voltage or an AC current that is applied between the respective electrodes, e.g. to obtain, at least in part, an impedance spectrum.

In another example of the present invention, a measure for an electrical capacitance of the sample may be determined from the second electrical signal, e.g. from the above mentioned electrochemical modelling of the electrochemical components between the electrodes used for the measurement of the second electrical signal.

The present invention also provides a device for determining the permeability of a sample through the observation of a diffusive process by electrochemical means. The devices comprises: (1) a first volume for holding a first medium; (2) a second volume for holding a second medium, the second volume being connected to the first volume; (3) a sample holder for mounting the sample such that the sample forms a barrier between the two volumes; (4) at least two electrodes in contact with the second volume; (5) a first measuring unit configured to measure a first electrical signal between at least two of the electrodes in contact with the second volume at different points in time, and (6) an electrical control unit configured to determine from said measured first electrical signal at the different points in time a first measure for the permeability of the sample, said first measure characterizing a permeability of the sample with respect to electrochemically active molecules contained in at least one of said first and second media.

The first and second volume can be formed or confined by walls of an impermeable material, e.g. glass, plastic / polymer, ceramic or metal, to hold a medium and the first and second volumes may be connected at a boundary surface. The sample holder is configured to hold a sample such that the sample separates the two volumes at the boundary surface as defined above.

The at least two electrodes in contact with the second volume can be enclosed completely by the second volume or can be located in the surrounding walls. The electrodes consist of an electrically conducting material, e.g. metal, semiconductor or conductive polymer, and are electrically connected to a first measuring unit.

The first measuring unit is configured to measure a first electrical signal between at least two of the electrodes in contact with the second volume. Said first measuring unit can comprise a voltmeter, e.g. a potentiometer, for measuring a voltage or an ammeter for measuring an electric current. The voltmeter can e.g. cover a range of about 1 Volt with a typical sensitivity of about 1 mV and the ammeter can have a range of about 10 mA with a typical sensitivity of about 0.1 $\mu$A. Furthermore, the first measuring unit can contain a voltage source for applying a voltage between at least two of the electrodes in contact with the second volume. The voltage source can be configured to apply different voltages between different pairs of electrodes. The supplied voltage can be a DC voltage or an AC voltage with typical amplitudes of about 1 to 100mV and typical frequencies of about 0.1Hz to 1MHz. The first measuring unit may contain a current source for inducing a predefined current between at least two of the electrodes in contact with the second volume. Additionally, the first measuring unit may be configured to determine the electrical impedance between the electrodes from the measured current/voltage and the applied voltage/current. The first measuring unit is configured to measure the first electrical signal at at least two different points in time and preferably to record a continuous or quasi-continuous time trace.

Finally, the device comprises an electrical control unit which can initiate a measurement by the first measuring unit and can receive the resulting data. The control unit determines from the measured first electrical signal the first measure for the sample's permeability, e.g. by extracting a rate of change of the first electrical signal or using a model for the diffusion process as described above. The first measure can for example be the molecular permeability coefficient or the diffusion constant of the sample with respect to the probe molecules. The control unit may be configured to provide the first measure to a user e.g. via a display or to a computer by an electronic interface like a USB port or a wireless connection.

The device can further comprise at least one electrode in contact with the first volume and a second measuring unit configured to measure a second electrical signal between at least one electrode in contact with the first volume and at least one electrode in contact with the second volume, The second measuring unit can be similar or identical to the first measuring unit and can also be connected to the electrical control unit, which may initiate a measurement by the second measuring unit and receive the resulting data. The first and second measuring units may be separate units, but could also be formed by a single unit providing both functionalities. The electrical control unit can be configured to determine from said second electrical signal a second measure for the permeability of the sample, said second measure characterizing, at least in part, a permeability of the sample with respect to ions, in particular inorganic ions, of an electrolyte solution forming at least one of said first and second media. The second measure can for example be the electrical resistance of the sample and can be determined as described above, e.g. from a DC electrical resistance or an AC impedance spectrum. The control unit can be configured to provide the second measure in the same way as the first measure.

The sample holder can be configured to support a sample which consists at least in part of biological cells. In particular, it can comprise a support membrane for providing mechanical support for one or multiple layers of biological cells. The support membrane can for example consist of a polymer, a metal, a ceramic, a semiconductor or a hydrogel permeable to electrochemically active molecules and electrolyte currents. Alternatively, the sample holder can be configured to carry ex vivo tissue samples,

As mentioned above, the first measuring unit can be configured to apply a voltage between at least two of said electrodes in contact with the second volume, in particular an AC voltage, and measure the resulting current between the electrodes, Similarly, the second measuring unit can be configured to apply a voltage between at least one electrode in contact with the first volume and at least one electrode in contact with the second volume, in particular an AC voltage, and measure the resulting current between the electrodes.

In an embodiment of the present invention, at least one of said first and second measuring units can be configured to determine a measure for an electrical impedance from the respective electrical signal. This measure can for example be a magnitude or phase of the electrical impedance or a combination thereof. Said at least one measuring unit may be configured to determine this measure at a single, fixed frequency of an AC voltage or an AC current that is applied between the respective electrodes or at a few, predefined frequencies. In particular, said at least one measuring unit may be configured to determine a measure for an electrical impedance as function of a frequency of an AC voltage or an AC current applied between at least two of the electrodes connected to the respective first or second measuring units.

In one example of the present invention, at least one electrode in contact with the first volume or in contact with the second volume can be a "large electrode", which has a comparatively large surface with a surface area deviating by less than 50%, preferably less than 25% from the surface area of the surface of the sample oriented towards the first or second volume. Here, the surface area of the sample is the corresponding base area and does not take into account the roughness of the sample surface. The large electrode surface can be parallel to the surface of the sample oriented towards the first or second volume or inclined by less than 10°, preferably less than 5°. A large surface area ensures a homogeneous current distribution throughout the sample if a voltage between a large electrode in contact with the first volume and a large electrode in contact with the second volume is applied to measure the second electrical signal. Such a homogeneous current distribution is required for a reliable measurement of the electrical resistance, see e. g. J. Wegener et al., Cell Tissue Res. 335, 485-514 (2014). Alternatively, the second measuring unit can be configured to apply the same voltage or current to at least two electrodes in contact with one of the volumes, thereby effectively creating a single electrode with an enlarged surface area.

At least two of the electrodes in contact with the same volume may have a combined surface area or shape covering more than 50%, preferably more than 75% of the surface area of the surface of the sample oriented towards the first or second volume. These at least two electrodes can be configured to be electrically connected to each other to form a combined electrode.

At least two of the electrodes in contact with the same volume can be interdigitated electrodes. Each of the interdigitated electrodes has a large perimeter in relation to its surface area and extents over a large area compared to its surface area. This facilitates a homogeneous detection over the entire area of the sample. The interdigitated electrodes are shaped such that the electrodes are interdigitated or interleaved. The gap between the at least two interdigitated electrodes may be similar along a large part of the perimeter of the electrodes. The perimeter of each of the interdigitated electrodes can be more than two times, preferably more than five times as large as the circumference of a circle with the same surface area as a surface of the electrode. The gap between two interdigitated electrodes can vary by at most 25%, preferably less than 10% over at least 50%, preferably 75% of the perimeter. The interdigitated electrodes may have the same surface area or surface areas that deviate by less than 25%, preferably less than 10% from each other. Furthermore, the interdigitated electrodes may be configured to form a combined electrode.

At least one of the electrodes in contact with the second volume can be a "small electrode", which has a surface area that is less than 10%, preferably less than 5% of the surface area of the sample oriented towards the second volume. Said at least one small electrode can be a single small electrode, which can be aligned to the center of the surface of the sample oriented towards the second volume. In particular, the small electrode can be at least partly surrounded by a large electrode.

In another example, said at least one small electrode can be a plurality of small electrodes, e.g. 3 to 10 electrodes, which can be distributed in a plane parallel to the surface of the sample oriented towards the second volume or inclined by less than 10°, preferably less than 5°. The plurality of small electrodes can be at least partly surrounded by a large electrode, and the first measuring unit can be configured to measure electrical signals between the large electrode and each one of the plurality of the small electrodes. This facilitates a local probing of the concentration of electrochemically active molecules in the vicinity of the small electrodes.

As mentioned above, said first measure may be a permeability coefficient of the sample, and said second measure may be an electrical resistance of the sample.

Finally, the sample holder can be removable from the device, e.g. for placing or mounting the sample onto the sample holder.

LIST OF FIGURES

In the following, a detailed description of the invention and exemplary embodiments thereof is given with reference to the figures. The figures show schematic illustrations of

Fig. 1a: a device for measuring the electrical resistance of a sample according to prior art

Fig. 1b: a device for determining a molecular permeability of a sample according to prior art

Fig. 2a: an example of an embodiment of the invention for an electrochemical determination of the molecular permeability of a sample

Fig. 2b: a further example of an embodiment of the invention for an electrochemical determination of the molecular permeability of a sample

Fig. 2c: a flow chart of a method to electrochemically determine the molecular permeability of a sample in accordance with an embodiment of the invention

Fig. 3a: an exemplary embodiment of the invention for a combined electrochemical determination of the molecular permeability and the electrical resistance of a sample

Fig. 3b: a flow chart of a method to electrochemically determine the molecular permeability and the electrical resistance of a sample in accordance with an embodiment of the invention

Fig. 4: an example of a sample holder for mounting biological cell membranes or biological cell layers according to an embodiment of the invention

Fig. 5a: an exemplary arrangement of a two-electrode setup according to an embodiment of the invention

Fig. 5b: an exemplary arrangement of a two-electrode setup with interdigitated electrodes according to an embodiment of the invention

Fig. 5c: an exemplary arrangement of a multi-electrode setup according to an embodiment of the invention

DESCRIPTION OF THE PREFERRED EMBODIMENTS

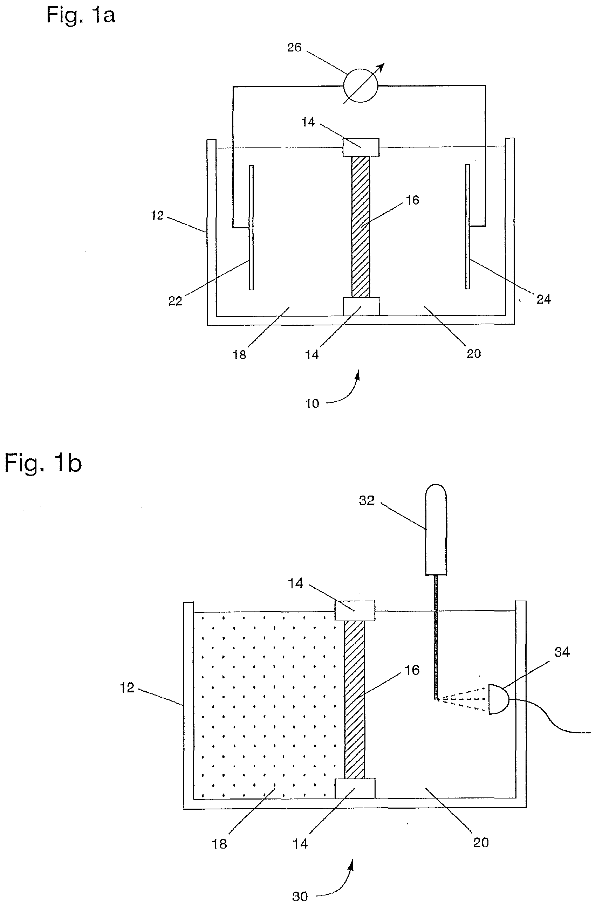

Fig. 1a illustrates a device 10 for measuring the electrical resistance of a sample as known from prior art. The device comprises a measurement chamber 12 and a sample holder 14, into which a sample 16 can be inserted such that it separates the measurement chamber into a first 18 and second volume 20 and forms a barrier in between. A first electrode 22 is located in the first volume 18 and a second electrode 24 in the second volume 20. The two electrodes 22 and 24 are connected via a measuring unit 26, which can measure an electrical signal between the electrodes 22 and 24. The measuring unit 26 can for example comprise a voltage source and an ammeter to measure the current between the electrodes 22 and 24 as a function of the applied voltage in order to determine the ohmic resistance of the sample 16 or its electrical impedance. To this end, the first 18 and second volume 20 are filled with a solution containing an electrolyte, which in conjunction with the sample 16 forms a conductive connection between the electrodes 22 and 24. The measuring unit 26 then applies a voltage to the electrodes 22 and 24 and measures the resulting current or its electrical impedance. If the resistance of the electrolyte solution as well as the other parts of the electrical circuit is known, the electrical resistance of the sample 16 can be calculated from this measurement.

In Fig. 1b, a setup 30 for determining the permeability of a sample 16 with respect to a molecular probe according to the prior art is shown. As in Fig. 1a, the sample 16 is placed in a sample holder 14, thereby separating the measurement chamber 12 into a first 18 and second volume 20.

Subsequently, a first medium is inserted into the first volume 18 and a second medium into the second volume 20 with the two media containing different concentrations of the probe molecules, illustrated as little black dots in Fig. 1b. Diffusion through the sample 16 enables the exchange of molecules between the two volumes 18 and 20 and the concentration gradient gives rise to a net current of molecules from the volume with a higher initial concentration to the volume with a lower initial concentration. This leads to an equilibration between the two volumes 18 and 20 such that the respective concentrations approach each other over time. The equilibration rate depends on the permeability of the sample 16 for the probe molecules and can thus be used to quantify the latter, e.g. by calculating the molecular permeability coefficient $P_E$ of the sample 16. The molecular permeability coefficient is given by the ratio of the diffusion constant of the probe molecules in the sample 16 and the thickness of the sample. Via Fick's law, the molecular permeability coefficient can be related to the exchange rate of molecules between the two volumes 18 and 20:

$$\frac{dN}{dt} = -P_E A\, \Delta c$$

where A is the surface of the sample 16 and $\Delta c$ the concentration difference between the volumes 18 and 20.

To measure the evolution of the concentration of molecules during the diffusion process, the molecules can for example be labeled by attaching a fluorophore, as disclosed in S. Sanders et al, Epithelial Cell Biol. 4, 25-34 (1995). The concentration can then be determined by optically exciting the fluorophore by a light source 32 like a laser or LED and detecting the resulting fluorescence with a photodetector 34, e.g. a photodiode or camera. Alternatively, other detection methods can be used, for example labeling the molecules by a radioactive substance and detecting the emitted radiation as described in H. Ghandehari et al., J. Pharm. Exp. Ther, 280, 747-753 (1997), or taking samples of the media at different points in time and subsequently analyzing them e.g. by chromatography, as described in K. Morimoto et al., Pharm. Res. 10, 1668-1674 (1993).

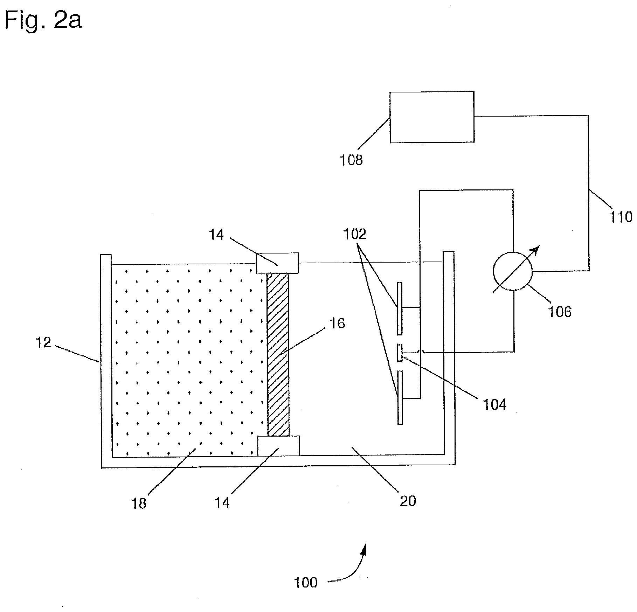

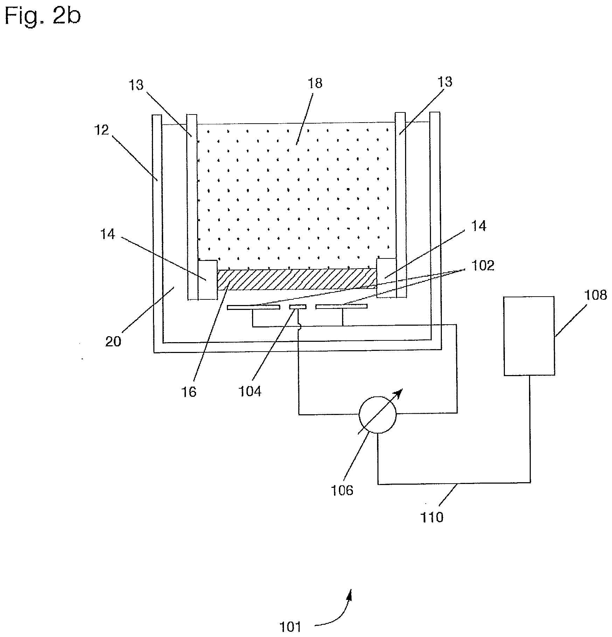

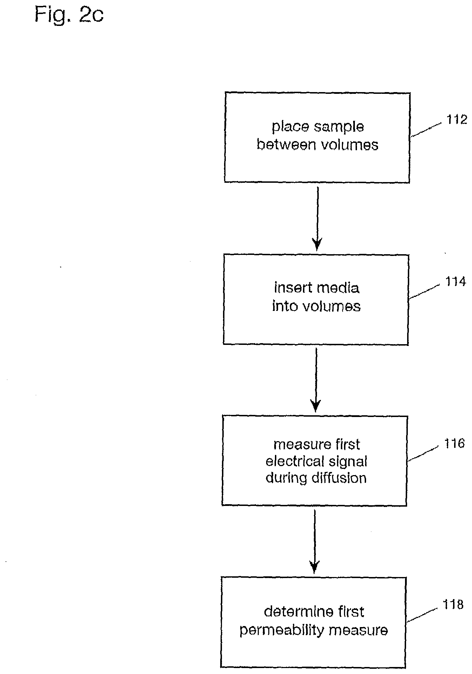

Fig. 2a shows an example for a device 100 according to an embodiment of the present invention, which can be used to implement a method as depicted in Fig. 2c. The device contains a measurement chamber 12 made of impermeable material like glass, which can for example form a rectangular box with an opening or a cylindrical laboratory beaker and is configured to hold a medium as defined below. The measurement chamber 12 comprises a sample holder 14, which holds a sample 16. The sample holder 14 can be removable from the measurement chamber 12 in parts or as a whole, e.g. to place or mount the sample 16 thereon. When mounted in or on the sample holder 14, the sample 16 separates the interior of the measurement chamber 12 into a first volume 18 and a second volume 20, forming a barrier in between as described above. The first 18 and the second volume 20 can be filled with a first and a second medium, which contain electrochemically active molecules in different concentrations as indicated by the small black dots in the first volume 18. The shape and arrangement of the two volumes 18, 20 and the sample 16 in Fig. 2a only constitute a specific example and many other layouts are possible. The sample 16 could for example be mounted horizontally such that the volumes 18, 20 are on top of each other (see below and Fig. 2b).

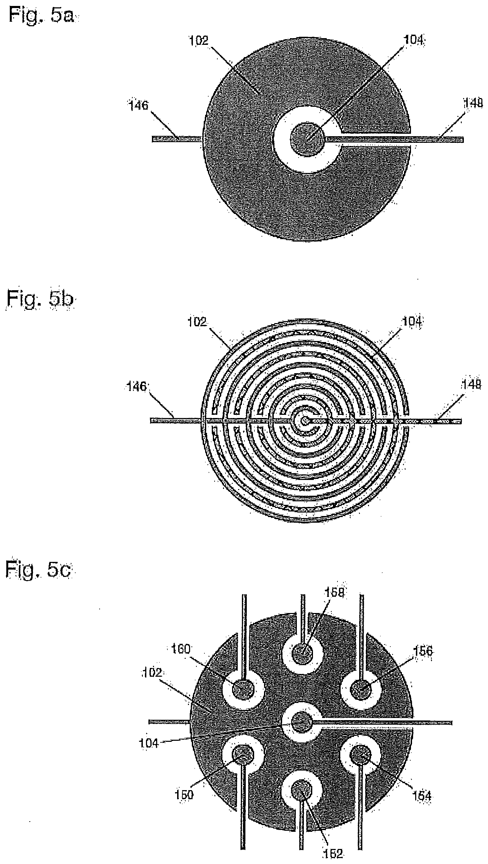

The device 100 includes a first electrode 102 and a second electrode 104 in contact with the second volume 20. The electrodes 102 and 104 are made of electrically conducting material and can either be located in the second volume 20 as shown in Fig. 2c, or can be integrated in the surrounding walls including bottom and top of the measurement chamber 12. Preferably, the electrodes 102, 104 are located close to the sample and the second volume 20 is smaller than the first volume 18 to facilitate the implementation of the method described below. The electrodes 102 and 104 can have different sizes and in particular, the first electrode 102 can be much larger than the second electrode 104, e.g. having a 100 times larger surface area. The first electrode 102 can at least partially surround the second electrode 104, which can be located in the center of the first electrode 102 (see Fig. 5a). The electrodes 102 and 104 can have at least one planar surface each, which is parallel to the surface of the sample 16 oriented towards the second volume 20 or inclined by less than 10°, preferably less than 5°.

The first 102 and second electrode 104 are electrically connected to a first measuring unit 106, which is configured to measure a first electrical signal between the electrodes 102, 104 at at least two different points in time. The first measuring unit 106 can comprise a voltage source configured to apply a DC or AC voltage to the electrodes 102, 104 and an ammeter to measure the current flowing between the electrodes 102, 104 in response to the applied voltage. The ammeter can be configured to record the amplitude of the current as well as its phase relative to the applied AC voltage. The amplitude of the voltage supplied by the voltage source can for example lie in the range of about 1 to 100mV with frequencies between 0.1 Hz and 1 MHz, which generates frequency-dependent currents with typical amplitudes of about 1 μA to 10 mA. The first measuring device 106 can be configured to vary the amplitude and frequency of the applied voltage during the measurement, e.g. to obtain an impedance spectrum or to determine the impedance at one or more fixed frequencies. In another example, the first measuring unit 106 can contain a voltmeter to measure a voltage between the electrodes 102, 104, in particular without inducing a current between the electrodes 102,104, e.g. a potentiometer. Typical specifications for the voltmeter are a range of about 1 V and a sensitivity of about 1 mV.

The first measurement unit 106 is connected to an electrical control unit 108 via a data bus 110 for the exchange of data. The electrical control unit 108 controls the measurement by the first measuring unit 106 and can set the measurement parameters like frequency and amplitude of the applied voltage and duration, number and spacing of data points. The data recorded by the first measuring unit 106 is transmitted to the electrical control unit 108, which is configured to store and analyze the data and determine the first permeability measure as discussed below with reference to Fig. 2c. For providing the first measure to a user, the electrical control unit 108 can comprise a display or similar means (not shown) as well as an electronic interface (not shown) like a USB port for a connection to an external device, e.g. a computer. This connection may be a wireless connection. The electronic interface can also be used to transfer the raw data or to externally control the measurement parameters. For the latter, the electronic control unit 108 can possess an input device like a touchscreen or switches and control knobs (not shown).

In Fig. 2b, another example for a device 101 according to an embodiment of the present invention is depicted, which can be used to implement a method as shown in Fig. 2c. In device 101, the sample 16 is mounted horizontally in the sample holder 14 and together with an interior wall 13 separates the interior of the measurement chamber 12 into a first volume 18 and a second volume 20. The interior wall 13 can consist of the same material as the measurement chamber 12. A horizontal orientation of the sample 16 can provide better mechanical support for the sample 16 and facilitate the mounting of the sample 16.

Fig. 2c is a flow diagram representing a method according to an embodiment of the present invention for determining a measure for the permeability of a sample relying on the diffusion of electrochemically active probe molecules through the sample and an observation of the evolution of their concentration by electrical means. Such a method can for example be implemented with the device 100 shown in Fig. 2a.

In a first step 112, the sample 16 is placed between a first volume 18 and a second volume 20 to form a barrier between the two volumes 18, 20. With the sample 16 in place, the two volumes 18, 20 are separated by the sample 16 in the sense that the sample 16 constitutes the predominant diffusive barrier between the volumes 18, 20. This can be achieved either by fully separating the two volumes 18, 20 such that they are not directly connected anymore or by partially separating them in such a way that the sample 16 is a limiting factor for an exchange of particles.

Subsequently, in step 114, a first medium is inserted into the first volume 18 and a second medium is inserted into the second volume 20. At least one of the first and second media contains at least one kind of electrochemically active probe molecules. The concentration of probe molecules can be zero in one of the media and nonzero in the other medium, or both media can contain probe molecules, but in different concentrations. It is not relevant for the method, which of the media contains the higher concentration of probe molecules. Preferably, the volumes of the first 18 and second volume 20 and the concentrations in the first 18 and second volume 20 are chosen such that the change in concentration in the second volume 20 during the equilibration between the volumes described below results in a sufficiently large change in the first electrical signal to be measured in step 116 (see below). In the context of the invention, the term electrochemically active molecules refers to all substances capable of creating an electrical signal between electrodes in contact with a medium containing the substance, wherein the electrical signal can be a voltage, a current. These substances include, but are not limited to, molecules and ions that can undergo oxidation processes or reduction processes, for example ferrocene methanol $C_{11}H_{12}FeO$, ferrocyanide $[Fe(CN)_6]^4$, ferricyanide $[Fe(CN)_6]^{3-}$, $[Ru(NH_3)_6]Cl_3$ or mixtures thereof. The probe molecules do not comprise any additional label like a fluorophore.

The first and second media can be any media in which the probe molecules can move freely, for example a liquid or a gel. The diffusion coefficient of the media with respect to the probe molecules should be larger than the one of the sample 16 to be probed in order for the transfer of particles between the media to be effectively restricted by the sample 16, Preferably, the media does not contain any kind of electrochemically active molecules other than the probe molecules to avoid unwanted contributions to the measurements and can e.g. be ultrapure water or a solution prepared in ultrapure water. For biological samples, the media can be cell culture media exhibiting an osmotic concentration suitable for the sample.

After the media are inserted into the respective volumes 18, 20, the electrochemically active molecules can diffuse through the sample 16, leading to an equilibration between the media as described above. During this diffusion process, in step 116, a first electrical signal is measured between at least two electrodes which are in contact with the second medium, e.g. electrodes 102, 104. This first electrical signal depends on the electrical properties of the second medium, which in turn depend on the concentration of electrochemically active molecules in the second medium. The first electrical signal thus serves as a probe to determine the probe molecule concentration during the equilibration. The signal can be measured at different discrete points in time or as a continuous time trace while the probe molecules diffuse through the sample 16.

The first electrical signal can for example be a voltage that builds up between two electrodes consisting of different materials in contact with the second medium due to the presence of probe molecules, e.g. electrodes 102, 104. The probe molecules can undergo an electrochemical reaction at at least one of the electrodes 102, 104 involving an electron transfer between the respective electrode and a probe molecule, thereby creating the voltage. The magnitude of the voltage depends on the concentration of probe molecules at the surfaces of the electrodes 102, 104.

The first electrical signal can also be a current, which arises for example when applying a voltage between two electrodes in contact with the second medium, e.g. electrodes 102, 104. The voltage difference gives rise to electrochemical reactions of the probe molecules at the surfaces of the electrodes 102, 104, e.g. a reduction process at one electrode and an oxidation process at the other electrode. The resulting transfer of electrons induces an electrical current between the electrodes 102, 104, which can be measured with an ammeter. For a given DC voltage, the current is determined by the charge transfer resistance at the surfaces of the electrodes 102, 104, which depends on the surface area and the concentration of probe molecules. Depending on the shape and position of the electrodes 102, 104 and choice of the probe molecules, this can enable the determination of the concentration at a well-defined position within the second volume 20. A calibration with known concentrations of probe molecules prior to the measurement allows for a quantitative determination of the concentration. The applied voltage can also be an AC voltage, in which case the induced AC current depends on the impedance between the electrodes 102, 104. As the electrodes 102, 104 are in contact with the second medium, the current does not pass through the sample 16, minimizing the influence of the measurement on the sample 16 and the diffusion process.

Information about the evolution of the concentration of probe molecules in the second volume 20 during the equilibration process is inferred from the first electrical signal in step 118. Based on this information, a first measure for the permeability of the sample 16 with respect to the probe molecules is determined. This first measure characterizes at least in part the permeability of the sample 16 for the electrochemically active molecules. A particularly simple example for this measure is the rate of change in the electrical signal between at least two points in time, which could additionally be normalized by the initial concentration difference between the first and second medium. If the electrical signal comprises many individual measurements or a time trace, the first measure can also be obtained using a model of the diffusion process, e.g. by fitting a fit function to the measured signal and extracting the first measure from the resulting fit parameters. The first measure could for example be the time constant of a fitted exponential function. This modelling can be particularly useful when more than one kind of electrochemically active molecule is contained in one or both of the media. Other examples for the first measure include the molecular permeability coefficient or diffusion constant of the sample 16 with respect to the probe molecules, which can be obtained by additionally taking into account geometric properties of the sample 16 like its surface area and width.

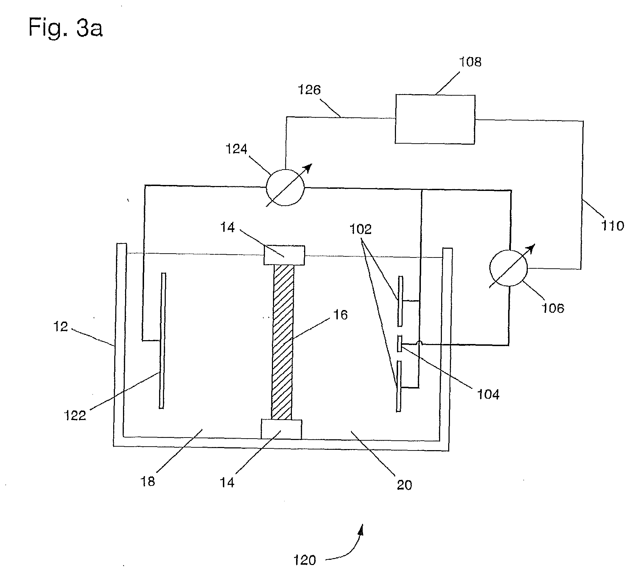

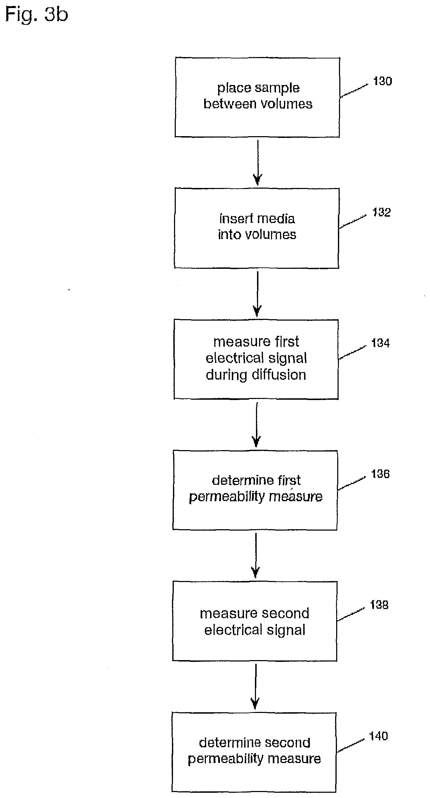

Fig. 3a depicts an example for a device 120 according to an embodiment of the present invention, which is configured to execute a method as the one illustrated in Fig. 3b and enables the determination of a first and second measure for the permeability of the sample 16 in the same experimental setup. The device 120 is an extension of the device 100 in Fig. 2a. In addition, the device 120 comprises at least one electrode in contact with the first volume 18, e.g. the electrode 122. The electrode 122 is connected to at least one electrode in contact with the second volume 20, in this case the electrode 102, via a second measuring unit 124. The second measuring unit 124 in turn is connected to the electrical control unit 108 via a data bus 126. Similar to device 101 in Fig. 2b, the sample 16 may also be mounted horizontally in other embodiments of the present invention.

The electrode 122 is made of electrically conducting material and can either be located in the first volume 18 as shown in Fig. 3b or can be integrated in the surrounding walls or bottom or top of the measurement chamber 12. The electrodes 122 and 102 can have a planar surface, which is parallel to the surface of the sample 16 oriented towards the first 18 or second volume 20 or inclined by less than 10°, preferably less than 5°. The electrodes 122 and 102 can further have a large surface, which has a surface area that is no less than 50%, preferably no less than 75% of the surface area of the sample 16 oriented towards the first 18 and second volume 20, respectively. The large surfaces can be planar surfaces and can be facing the sample 16. When applying a voltage between the electrodes 122 and 102, a surface area comparable to the surface area of the sample 16 creates a homogeneous distribution of electric fields and currents and thereby enables a reliable measurement of the sample properties and reduces stress on the sample 16.

The second measuring unit 124 can be similar or identical to the first measuring unit 106. In particular, it can be configured to apply a DC or AC voltage between the electrodes 122 and 102 and to measure the current flowing between the electrodes 122, 102, e.g. as a function of the frequency of the voltage. Furthermore, the first 106 and second measuring unit 124 could also be formed by a single unit providing both functionalities.

Similar to the first measuring unit 106, the second measuring unit 124 and the electrical control unit 108 are connected by a data bus 126 for data exchange and the second measuring unit 124 is controlled by the electrical control unit 108. The electrical control unit 108 is designed to analyze the data obtained from the second measuring unit 124 and determine the second permeability measure e.g. as in step 140 of Fig. 3b.

In Fig. 3b, another example of a method for determining the permeability of a sample according to an embodiment of the present invention is illustrated. This method can for example be implemented with the device 120 depicted in Fig. 3a. The first four steps 130-136 are equivalent to the corresponding steps 112-118 of the method shown in Fig. 2a to obtain a first measure of the sample 16 with respect to the probe molecules. The method in Fig. 3b further comprises the measurement, step 138, of a second electrical signal between at least one electrode in contract with the first medium and at least one electrode in contact with the second medium, e.g. electrodes 122 and 102, wherein at least one of the media is an electrolyte solution. From this second electrical signal, a second measure for the permeability of the sample 16 for the ions of said electrolyte is determined in step 140, This method facilitates the determination of a first and second measure for the permeability of a sample 16 with similar techniques and in a single experiment without requiring a transfer of the sample 16 between different experimental setups or the use of different samples.

The second electrical signal depends on the electrical conductivity of the sample 16 for an electrolyte current and can for example be an electric current in response to a voltage applied to an electrode 122 in the first volume 18 and an electrode 102 in the second volume 20. This voltage creates a potential difference for the charged ions of the electrolyte across the sample 16 and thus leads to a directional motion of ions through the sample 16, which depends on the permeability of the sample 16 with respect to the ions. The applied voltage can be a DC voltage to determine the DC electrical resistance between the electrodes 122 and 102, or it can be an AC voltage to determine the impedance. The frequency of the AC voltage can be fixed or it may be varied during the measurement, e.g. to study the response of the system as a function of the frequency. As the voltage difference and current flow occur at least in part across the sample 16, special care has to be taken to avoid perturbing the intrinsic diffusive properties of the sample 16 or even damaging the sample 16, e.g. by choosing sufficiently small voltages.

The measurement of the second electrical signal in step 138 is preferably conducted with the first and second medium in or close to equilibrium such that they contain the same concentration of ions. In this way, an additional contribution to the electrolyte current arising from a concentration gradient driven diffusion across the sample 16 can be prevented. Alternatively, the magnitude and/or frequency of the current occurring during the measurement of the second electrical signal can be chosen such that the effect of the diffusion on the measurement is negligible or that the contributions can be isolated.

The ions of the electrolyte may be or contain the electrochemically active molecules, in which case the first measure characterizes at least in part an unperturbed diffusive permeability of the sample 16 for the electrochemically active molecules, whereas the second measure characterizes at least in part an externally driven permeability in the presence of an external electric field across the sample 16. In this case, the measurement of the second electrical signal 138 should be conducted after the measurement of the first electrical signal 134 and when the first and second media have at least nearly equilibrated, i.e. the concentrations of electrochemically active molecules have approached each other. This prevents a modification of the diffusion process due to a voltage difference across the sample 16. In another example, the magnitude and/or frequency of the current occurring during the measurement of the second electrical signal as well as the duration of the measurement can be chosen such that the effect of the measurement on the diffusion process is negligible. This enables e.g. a measurement of the first and second electrical signal in an alternating fashion during the equilibration.

Alternatively, the ions of the electrolyte can be different from the electrochemically active molecules. The ions can have a different size than the electrochemically active molecules and can in particular be small inorganic ions, Furthermore, the concentration of ions in the first and second medium can be the same to minimize the influence of the ions on the measurement of the first electrical signal 134 during the diffusion of the electrochemically active molecules. The measurement of the second electrical signal in 138 can be performed during or after the equilibration process as described above. This requires, however, a careful analysis of the effect that the electrochemically active molecules have on the second electrical signal. To avoid this, a concentrations of ions and/or electrochemically active molecules can be used for which the contribution of the electrochemically active molecules on the second electrical signal is negligible. In one example, the concentration of ions can be about 100 times larger than the concentration of electrochemically active molecules. As described above, if the measurement of the second electrical signal is conducted during the equilibration, the influence on the diffusion process can be reduced by an appropriate choice of the magnitude and/or frequency of the currents and voltages involved and the duration of the measurement.

In another example, the order of the two measurements 134 and 138 can be reversed and the method can be modified such that the first and second media initially contain the same concentration of ions, but no electrochemically active molecules. The second electrical signal is then recorded in step 138 after inserting the media into the volumes 18, 20 and the second permeability measure is determined from this, in 140. Subsequently, the electrochemically active molecules are added to one of the media, initiating the diffusion process during which the first electrical signal is measured, in 134. Due to the homogeneous and constant concentration of ions, the contribution of the ions to the first electrical signal should amount to a constant offset and thus not affect the determination of the first permeability measure for the electrochemically active molecules in step 136.

In step 140, a second measure for the permeability of the sample 16 is determined based on the second electrical signal. This second measure characterizes at least in part a permeability of the sample 16 with respect to the ions of the electrolyte. The second measure can for example be the electrical resistance of the sample 16, which can be obtained by comparing the measured DC electrical resistance between the electrodes 122 and 102 with the corresponding quantity measured without a sample 16 mounted in the sample holder 14 in identical media. The second measure can also be an impedance spectrum or quantities extracted from an impedance spectrum, e.g. the electrical resistance and the capacitance of the sample 16. The latter can allow for the characterization of additional properties of the sample 16 like the surface area or dielectric constant. Step 140 can also include a modelling of the second electrical signal and in particular an impedance spectrum obtained from the second electrical signal. The model can be an electrochemical model, which can for example account for effects like the impedance of the sample holder 14 and the media or the charge transfer resistance of the electrodes 122,102. The second measure can be a fit parameter of a fit to the second electrical signal or to an impedance spectrum. From the impedance spectrum of the sample 16, additional information about the diffusion process may be obtained, e.g. the relevant types of transport like paracellular or transcellular transport as well as their individual contributions, cf. D. Günzel et al., Curr. Top. Membr. 65,39-78 (2010).

The order of the steps in Fig. 3b is only an exemplary illustration and can be different in other examples of the present invention. As already discussed above, the order of the measurements in 134 and 138 can be reversed. In another example, the determination of the first permeability measure in 136 can be performed after the measurement of the second electrical signal 138, e.g. simultaneously with the determination of the second measure in 140. Moreover, the measurements of the first and second electrical signal can be conducted in an alternating way, wherein the first and second electrical signal are measured sequentially and these measurements are repeated multiple times, e.g. to obtain a time trace or using different measurement parameters. Alternatively, the signals can be measured simultaneously, e.g. at different frequencies to allow for a separation of the relevant contributions.

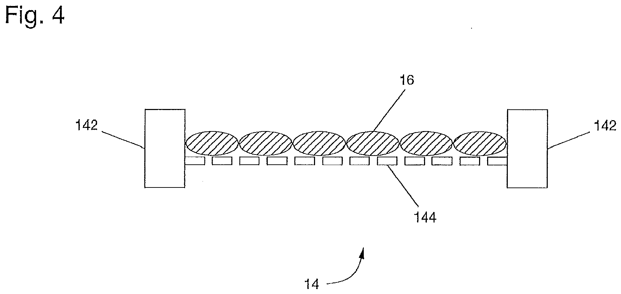

Fig. 4 shows an example of a sample holder 14 for the mounting of a sample 16 containing biological cells and in particular samples consisting of a single or multiple layers of biological cells. The sample holder 14 comprises an outer mount 142 for mounting the sample holder 14 in a measurement chamber 12 e.g. in devices 100 or 120. The sample holder 14 can be designed such that the sample 16 is oriented vertically as shown in Figs. 2a and 3a. In other examples, the sample holder 14 can be configured such that the sample 16 is mounted horizontally in the measurement chamber 12 as shown in Fig. 2b. The outer mount 142 consists of an impermeable material and is designed to fit in the measurement chamber 12 such that the sample 16 held between the outer mount 142 separates the measurement chamber 12 into a first 18 and second volume 20 and constitutes the main diffusive connection between the two volumes 18 and 20, whereas the outer mount 142 seals off the remainder of the boundary between the two volumes 18 and 20. The outer mount 142 can be removable from the measurement chamber 12. Attached to the outer mount 142 is a support membrane 144, which can span the entire area enclosed by the outer mount 142. The support membrane 144 is configured to provide mechanical support for the sample 16, which can for example be a membrane consisting of one or more layers of biological cells as illustrated in Fig. 4. The sample 16 is mounted to the support membrane 144 in such a way that the sample 16 remains attached to the support membrane 144 even when surrounded by a media as used in devices 100 and 120, in particular if the sample 16 is oriented vertically as shown in Figs. 2a and 3a. The support membrane 144 can consist of a polymer, porous glass or porous semiconductor material, which should exhibit a high permeability with respect to the electrochemically active molecules and ions used in the experiments. The support membrane 144 can be removable from the outer mount 16, e.g. for placing or seeding cells 16 onto the support membrane 144. The latter can for example be done by directly growing a cell layer 16 on the support membrane 144 in a cell culture. In other examples, it may be sufficient to place the sample 16 loosely on the support membrane 144, e.g. if the sample 16 is oriented horizontally when mounted in the measurement chamber 12.

In Fig. 5a, an exemplary design for the two electrodes 102 and 104 in contact with the second volume 20 is illustrated in top view as seen from the position of the sample 16. The electrode 102, which is employed in the measurement of both the first and the second electrical signal, is larger than the electrode 104 and constitutes the counter electrode to the electrode 104 when measuring the first electrical signal. The smaller electrode 104 is sometimes also referred to as working electrode. Both electrodes 102 and 104 can be contacted via a small wire attached to them, 146 and 148, respectively. As described above, the surface area of the electrode 102 can be similar to the surface area of the sample 16 oriented towards the second volume 20. The electrode 104 can be a small electrode, which has a surface area that is less than 10%, preferably less than 5% of the surface area of the sample 16 oriented towards the second volume 20. For a suitable choice of electrochemically active molecules, e.g. with a positive or negative charge, and of the applied voltage, this electrode design can facilitate a measurement of a first electrical signal, which depends mostly on the concentration of probe molecules in the vicinity of the small electrode 104. This can provide a local measure of the concentration and thus the permeability of the sample 16. The electrode 104 can be located in the center of the electrode 102. Both electrodes 102 and 104 can have a planar surface and lie in the same plane, e.g. parallel to the surface of the sample 16 oriented towards the second volume 20 or inclined by less than 10°, preferably less than 5°.

Fig. 5b depicts another example of a two-electrode setup, e.g. for the two electrodes 102 and 104 in contact with the second volume 20. In this example, the electrodes 102, 104 are interdigitated electrodes. The shape of the electrodes 102, 104 is designed in order to achieve a similar gap between the electrodes 102, 104 at different points, while at the same time covering a large area without obstructing the diffusion into or out of the volume in the immediate vicinity of the electrodes 102, 104. This facilitates for example a reliable determination of the average concentration of electrochemically active molecules in the second volume 20 via a measurement of the first electrical signal between electrodes 102, 104. The electrodes 102, 104 can be configured to be electrically connected to each other, e.g. during the measurement of the second electrical signal, to form a combined electrode extending over a large area.

The devices 100 and 120 can contain a plurality of electrodes in contact with the second volume 20 instead of the two electrodes 102 and 104 as shown in Fig. 2a. Similarly, device 120 can also have a plurality of electrodes in contact with the first volume 18. An example for a corresponding electrode layout is shown in Fig. 5c. It comprises a large electrode 102, which can for example be used for the measurement of the second electrical signal. In addition, seven small electrodes 104 and 150-160 are distributed over the area covered by the electrode 102, which can correspond to the size of the sample 16.

The first electrical signal could for example be measured between one small electrode, e.g. electrode 104 and the large electrode 102. Additional electrical signals can be measured in a pairwise fashion between the small electrodes 150-160 and the large electrode 102 as the counter electrode, e.g. a third electrical signal is measured between the small electrode 150 and the electrode 102, a fourth electrical signal between electrodes 152 and 102 and so on. This can enable a local probing of the evolution of the probe molecule concentration during the diffusion process and thereby provide spatially resolved information about the permeability of the sample 16 with respect to the electrochemically active molecules. For this, a third measure for the permeability can be determined from the third electrical signal and correspondingly an additional measure for each additional electrical signal that is measured. By combining the different measures as well as information about the location of the respective electrodes, the spatial distribution of the permeability can be mapped out.

Information about the spatial distribution not only allows for the characterization of the permeability of inhomogeneous samples, but can also be used to assess the quality of the sample 16. In particular, this information can allow identifying defective samples with defects like holes or impurities, which affect the permeability. This provides a simple tool to determine the general validity of the obtained permeability measures for the substance or material that the sample 16 consists of. This holds in particular in combination with the measurement of the second electrical signal and the determination of the second permeability measure. The method according to an embodiment of the present invention can for example be extended by obtaining a measure for the homogeneity of the sample 16 from this information, e.g. the standard deviation of the measures obtained from the electrical signals between the electrodes 102, 104 and 150-160 in contact with the second volume 20. This measure can then be compared with one or more predefined criteria characterizing the desired minimum homogeneity, e.g. an upper threshold for the standard deviation. This comparison can be used to decide whether the measurements should be repeated with a different sample if the present sample 16 is suspected to be damaged or otherwise compromised.

The electrode designs in Fig. 5 are only examples of potential layouts. Many other implementations with different shapes and positions of the electrodes are possible. Furthermore, the number of electrodes can be different. A multi-electrode array can be used in both the first 18 and second volume 20, which can for example also allow for a local determination of the permeability of the sample 16 with respect to the ions of the electrolyte.

The embodiments of the present invention disclosed herein only constitute specific examples for illustration purposes. The present invention can be implemented in various ways and with many modifications without altering the underlying basic properties. Therefore, the present invention is only defined by the claims as stated below.

LIST OF REFERENCE SIGNS

| | |
|---|---|
| 10 | - Device for measuring the electrical resistance of a sample |
| 12 | - Measurement chamber |
| 13 | - interior wall |
| 14 | - Sample holder |
| 16 | - Sample |
| 18 | - First volume |
| 20 | - Second volume |
| 22 | - Electrode |
| 24 | - Electrode |
| 26 | - Measuring unit |
| 30 | - Device for measuring the molecular permeability of a sample |
| 32 | - Light source |
| 34 | - Photodetector |
| 100 | - Device for an electrochemical determination of the molecular permeability of a sample |
| 101 | - Device for an electrochemical determination of the molecular permeability of a sample |
| 102 | - First electrode in contact with the second volume 20 |
| 104 | - Second electrode in contact with the second volume |
| 106 | - First measuring unit |
| 108 | - Electrical control unit |
| 110 | - Data bus |
| 112 | - Step of placing the sample 16 between the first 18 and second volume 20 |
| 114 | - Step of inserting a first and second medium into the first 18 and second volume 20 |
| 116 | - Step of measuring the first electrical signal |
| 118 | - Step of determining the first permeability measure |
| 120 | - Device for a combined electrochemical determination of the molecular permeability and the electrical resistance of a sample |
| 122 | - Electrode in contact with the first volume 18 |
| 124 | - Second measuring unit |
| 126 | - Data bus |
| 130 | - Step of placing the sample 16 between the first 18 and second volume 20 |
| 132 | - Step of inserting a first and second medium into the first 18 and second volume 20 |
| 134 | - Step of measuring the first electrical signal |
| 136 | - Step of determining the first permeability measure |
| 138 | - Step of measuring the second electrical signal |
| 140 | - Step of determining the second permeability measure |

142    - Outer mount
144    - Support membrane
146    - Connecting wire
148    - Connecting wire

150-160    - Electrodes

## Claims

1. A method for determining the permeability of a sample (16) through observation of a diffusive process by electrochemical means, the method comprising the following steps:

   placing the sample (16) between a first (18) and a second volume (20) to form a barrier between the two volumes (18, 20),
   providing a first medium in the first volume (18) and a second medium in the second volume (20), wherein at least one of the first and second media contains at least one kind of electrochemically active molecules, and wherein the concentrations of said electrochemically active molecules in said first and second media are different from each other,
   measuring a first electrical signal between at least two electrodes (102, 104) in contact with the second volume (20) at different points in time while the electrochemically active molecules are allowed to diffuse through the sample (16), said first electrical signal depending on the concentration of electrochemically active molecules at at least one position in the second volume (20),
   determining from said measured first electrical signal at the different points in time a first measure for the permeability of the sample (16), said first measure characterizing a permeability of the sample (16) with respect to said electrochemically active molecules.

2. The method of Claim 1, wherein at least one of said first and second media is an electrolyte solution, and wherein the method further comprises the following steps:

   measuring a second electrical signal between at least one electrode (122) in contact with the first volume (18) and at least one electrode (102) in contact with the second volume (20), said second electrical signal depending on the electrical conductivity of the sample (16) with respect to an electrolyte current, and
   determining from said second electrical signal a second measure for the permeability of the sample (16), said second measure characterizing, at least in part, a permeability of the sample (16) with respect to ions, in particular inorganic ions, of said electrolyte.

3. The method according to Claim 2, wherein the first and second media initially do not contain said electrochemically active molecules and said electrochemically active molecules are added to at least one of the media after the measurement of the second electrical signal and before the measurement of the first electrical signal.

4. The method according to any one of the preceding claims, wherein the sample (16) consists, at least in part, of biological cells, and/or
   wherein said first measure is a permeability coefficient of the sample (16), and/or
   wherein said second measure is an electrical resistance of the sample (16).

5. The method according to any one of the preceding claims, further comprising the following steps:

   measuring at least a third electrical signal between at least two electrodes (102, 150) in contact with the second volume (20) at different points in time while the electrochemically active molecules are allowed to diffuse through the sample (16), said third electrical signal depending on the concentration of electrochemically active molecules at at least one position in the second volume (20), wherein at least one of said at least two electrodes (102,150) is different from the at least two electrodes (102, 104) used for the measurement of said first electrical signal,
   determining from said third electrical signal a third measure for the permeability of the sample (16), said third measure characterizing, at least in part, a permeability of the sample (16) with respect to said electrochemically active molecules.

6. The method according to Claim 5, further comprising determining from said first and said at least third electrical signal information pertaining the spatial distribution of a permeability of the sample (16) with respect to said electrochemically active molecules, wherein the method preferably further comprises obtaining a measure for the homogeneity of the sample (16) from said information pertaining the spatial distribution of a permeability, comparing said homogeneity measure to predefined criteria characterizing the homogeneity of the sample (16), and repeating the measurements with a new sample if said criteria are not met.

7. The method according to any one of the preceding claims, wherein at least one of the two media is a fluid, in particular a liquid, or a gel, and/or

wherein the transfer of said electrochemically active molecules through the sample (16) involves paracellular transport.

8. The method according to any one of Claims 2-7, wherein the transfer of ions of said electrolyte through the sample (16) involves paracellular transport, and/or
wherein said electrochemically active molecules are larger than the ions of said electrolyte, and/or wherein the method
further comprises determining from said second electrical signal a measure for an electrical capacitance of the sample (16).

9. The method according to any one of the preceding claims, wherein at least one of said electrical signals is measured by applying a voltage between at least two of said electrodes (102, 104, 122, 150), in particular an AC voltage, and measuring the resulting current between the electrodes, and/or wherein the method
further comprises determining from at least one of said first and second electrical signals a measure for an electrical impedance, and in particular a measure for an electrical impedance as a function of a frequency of an AC voltage or an AC current applied between the at least two electrodes used for the measurement of the respective first or second electrical signal.

10. A device (100) for determining the permeability of a sample (16) through observation of a diffusive process by electrochemical means, the device comprising:

a first volume (18) for holding a first medium,
a second volume (20) for holding a second medium, the second volume (20) being connected to the first volume (18),
a sample holder (14) for mounting the sample (16) such that the sample (16) forms a barrier between the two volumes (18,20),
at least two electrodes (102, 104) in contact with the second volume (20),
a first measuring unit (106) configured to measure a first electrical signal between at least two (102, 104) of the electrodes in contact with the second volume (20) at different points in time,
an electrical control unit (108) configured to determine from said measured first electrical signal at the different points in time a first measure for the permeability of the sample (16), said first measure characterizing a permeability of the sample (16) with respect to electrochemically active molecules contained in at least one of said first and second media.

11. The device (100) of Claim 10, further comprising:

at least one electrode (122) in contact with the first volume (18),
a second measuring unit (124) configured to measure a second electrical signal between at least one electrode (122) in contact with the first volume (18) and at least one electrode (102) in contact with the second volume (20),
wherein the electrical control unit (108) is configured to determine from said second electrical signal a second measure for the permeability of the sample (16), said second measure characterizing, at least in part, a permeability of the sample (16) with respect to ions, in particular inorganic ions, of an electrolyte solution forming at least one of said first and second media.

12. The device according to Claim 10 or 11, wherein the sample holder (14) is configured to support a sample (16) which consists at least in part of biological cells, and is in particular formed by a support membrane (144) for supporting one or multiple layers of biological cells, and/or
wherein the first measuring unit (106) is configured to apply a voltage between at least two (102, 104) of said electrodes in contact with the second volume (20), in particular an AC voltage, and measure the resulting current between the electrodes (102, 104), and/or
wherein the second measuring unit (124) is configured to apply a voltage between at least one electrode (122) in contact with the first volume (18) and at least one electrode (102) in contact with the second volume (20), in particular an AC voltage, and measure the resulting current between the electrodes (102, 122), and/or
wherein at least one of said first and second measuring units (106, 124) is configured to determine a measure for an electrical impedance, and in particular a measure for an electrical impedance as function of a frequency of an AC voltage or an AC current applied between at least two of the electrodes (102, 104, 122) connected to the respective first or second measuring unit (106, 124).

13. The device according to any one of the preceding Claims 10-12, wherein a surface of at least one of the electrodes (102, 104, 122) in contact with the first (18) or second volume (20) is parallel to the surface of the sample (16) oriented towards the first (18) or second volume (20) or inclined by less than 10°, preferably less than 5° and has a surface area deviating by less than 50%, preferably less than 25% from the surface area of the surface of the sample (16) oriented towards the first (18) or second volume (20), and/or
wherein at least two of the electrodes in contact with the same volume are interdigitated electrodes, each of which has a perimeter that is more than two times, preferably more than five times as large as the circumference of a circle with the same surface area as a surface of the electrode.

14. The device according to any one of the Claims 10-13, wherein at least one of the electrodes (102, 104) in contact with the second volume (20) is a small electrode, which has a surface area that is less than 10%, preferably less than 5% of the surface area of the sample (16) oriented towards the second volume (20),

wherein said at least one small electrode is preferably a single small electrode (104), which is aligned to the center of the surface of the sample (16) oriented towards the second volume (20), or

wherein said at least one small electrode is preferably a plurality of small electrodes (104, 150-160), which are distributed in a plane that is parallel to the surface of the sample (16) oriented towards the second volume (20) or inclined by less than 10°, preferably less than 5°.

15. The device according to any one of the preceding Claims 10-14, wherein said first measure is a permeability coefficient of the sample (16), and/or

wherein said second measure is an electrical resistance of the sample (16), and/or

wherein the sample holder (14) is removable from the device.

**Patentansprüche**

1. Verfahren zur Bestimmung der Permeabilität einer Probe (16) durch Beobachtung eines Diffusionsprozesses mit elektrochemischen Mitteln, wobei das Verfahren die folgenden Schritte umfasst:

Anordnen der Probe (16) zwischen einem ersten (18) und einem zweiten Volumen (20), um eine Barriere zwischen den beiden Volumen (18, 20) zu bilden,

Bereitstellen eines ersten Mediums in dem ersten Volumen (18) und eines zweiten Mediums in dem zweiten Volumen (20), wobei mindestens eines des ersten und des zweiten Mediums mindestens eine Art von elektrochemisch aktiven Molekülen enthält und wobei die Konzentrationen der elektrochemisch aktiven Moleküle in dem ersten und dem zweiten Medium voneinander verschieden sind,

Messen eines ersten elektrischen Signals zwischen mindestens zwei Elektroden (102, 104), die mit dem zweiten Volumen (20) in Kontakt sind, zu verschiedenen Zeitpunkten, während die elektrochemisch aktiven Moleküle durch die Probe (16) diffundieren können, wobei das erste elektrische Signal von der Konzentration der elektrochemisch aktiven Moleküle an mindestens einer Position in dem zweiten Volumen (20) abhängt,

Bestimmen eines ersten Maßes für die Permeabilität der Probe (16) aus dem zu den verschiedenen Zeitpunkten gemessenen ersten elektrischen Signal, wobei das erste Maß eine Permeabilität der Probe (16) in Bezug auf die elektrochemisch aktiven Moleküle charakterisiert.

2. Verfahren nach Anspruch 1, wobei mindestens eines der ersten und zweiten Medien eine Elektrolytlösung ist, und wobei das Verfahren ferner die folgenden Schritte umfasst:

Messen eines zweiten elektrischen Signals zwischen mindestens einer Elektrode (122), die mit dem ersten Volumen (18) in Kontakt steht, und mindestens einer Elektrode (102), die mit dem zweiten Volumen (20) in Kontakt steht, wobei das zweite elektrische Signal von der elektrischen Leitfähigkeit der Probe (16) in Bezug auf einen Elektrolytstrom abhängt, und

Bestimmen eines zweiten Maßes für die Permeabilität der Probe (16) aus dem zweiten elektrischen Signal, wobei das zweite Maß zumindest teilweise eine Permeabilität der Probe (16) in Bezug auf Ionen, insbesondere anorganische Ionen, des Elektrolyten charakterisiert.

3. Verfahren nach Anspruch 2, wobei das erste und das zweite Medium anfänglich nicht die elektrochemisch aktiven Moleküle enthalten und die elektrochemisch aktiven Moleküle zu mindestens einem der Medien nach der Messung des zweiten elektrischen Signals und vor der Messung des ersten elektrischen Signals zugegeben werden.

4. Verfahren nach einem der vorhergehenden Ansprüche, wobei die Probe (16) zumindest teilweise aus biologischen Zellen besteht, und/oder

wobei das erste Maß ein Permeabilitätskoeffizient der Probe (16) ist, und/oder

wobei das zweite Maß ein elektrischer Widerstand der Probe (16) ist.

5. Verfahren nach einem der vorhergehenden Ansprüche, ferner umfassend die folgenden Schritte:

Messen mindestens eines dritten elektrischen Signals zwischen mindestens zwei Elektroden (102,150), die mit dem zweiten Volumen (20) in Kontakt stehen, zu unterschiedlichen Zeitpunkten, während die elektrochemisch aktiven Moleküle durch die Probe (16) diffundieren können, wobei das dritte elektrische Signal von der Konzentration der elektrochemisch aktiven Moleküle an mindestens einer Position in dem zweiten Volumen (20) abhängt, wobei mindestens eine der mindestens zwei Elektroden (102, 150) sich von den mindestens zwei Elektroden (102, 104) unterscheidet, die für die Messung des ersten elektrischen Signals verwendet wurden, Bestimmen eines dritten Maßes für die Permeabilität der Probe (16) aus dem dritten elektrischen Signal, wobei das dritte Maß zumindest teilweise eine Permeabilität der Probe (16) in Bezug auf die elektrochemisch aktiven Moleküle charakterisiert.

6. Verfahren nach Anspruch 5, ferner umfassend Bestimmen von Informationen aus dem ersten und dem mindestens dritten elektrischen Signal, die die räumliche Verteilung einer Permeabilität der Probe (16) in Bezug auf die elektrochemisch aktiven Moleküle betreffen, wobei das Verfahren vorzugsweise ferner folgendes umfasst
Gewinnen eines Maßes für die Homogenität der Probe (16) aus der Information, die die räumliche Verteilung einer Permeabilität betrifft,
Vergleichen des Homogenitätsmaßes mit vordefinierten Kriterien, die die Homogenität der Probe (16) charakterisieren, und
Wiederholen der Messungen mit einer neuen Probe, wenn die Kriterien nicht erfüllt sind.

7. Verfahren nach einem der vorhergehenden Ansprüche, wobei mindestens eines der beiden Medien ein Fluid, insbesondere eine Flüssigkeit, oder ein Gel ist, und/oder
wobei der Transfer der elektrochemisch aktiven Moleküle durch die Probe (16) einen parazellulären Transport beinhaltet.

8. Verfahren nach einem der Ansprüche 2-7, wobei der Transfer von Ionen des Elektrolyten durch die Probe (16) einen parazellulären Transport beinhaltet, und/oder
wobei die elektrochemisch aktiven Moleküle größer sind als die Ionen des Elektrolyten, und/oder wobei das Verfahren
ferner das Bestimmen eines Maßes für eine elektrische Kapazität der Probe (16) aus dem zweiten elektrischen Signal umfasst.

9. Verfahren nach einem der vorhergehenden Ansprüche, wobei mindestens eines der elektrischen Signale durch Anlegen einer Spannung zwischen mindestens zwei der Elektroden (102,104,122,150), insbesondere einer Wechselspannung, und Messen des resultierenden Stroms zwischen den Elektroden gemessen wird, und/oder wobei das Verfahren weiterhin das Bestimmen eines Maßes für eine elektrische Impedanz aus mindestens einem des ersten und zweiten elektrischen Signals umfasst, und insbesondere ein Maß für eine elektrische Impedanz als eine Funktion einer Frequenz einer Wechselspannung oder eines Wechselstroms, die bzw. der zwischen den mindestens zwei für die Messung des jeweiligen ersten oder zweiten elektrischen Signals verwendeten Elektroden angelegt wird.

10. Vorrichtung (100) zur Bestimmung der Permeabilität einer Probe (16) durch Beobachtung eines Diffusionsprozesses mit elektrochemischen Mitteln, wobei die Vorrichtung folgendes umfasst:

ein erstes Volumen (18) zur Aufnahme eines ersten Mediums,
ein zweites Volumen (20) zur Aufnahme eines zweiten Mediums, wobei das zweite Volumen (20) mit dem ersten Volumen (18) verbunden ist,
einen Probenhalter (14) zum Befestigen der Probe (16), so dass die Probe (16) eine Barriere zwischen den beiden Volumen (18, 20) bildet,
mindestens zwei Elektroden (102, 104), die in Kontakt mit dem zweiten Volumen (20) stehen,
eine erste Messeinheit (106), die so konfiguriert ist, dass sie ein erstes elektrisches Signal zwischen mindestens zwei (102, 104) der Elektroden, die mit dem zweiten Volumen (20) in Kontakt sind, zu verschiedenen Zeitpunkten misst,
eine elektrische Steuereinheit (108), die dazu eingerichtet ist, aus dem zu den verschiedenen Zeitpunkten gemessenen ersten elektrischen Signal ein erstes Maß für die Permeabilität der Probe (16) zu bestimmen, wobei das erste Maß eine Permeabilität der Probe (16) in Bezug auf elektrochemisch aktive Moleküle charakterisiert, die in mindestens einem aus dem genannten ersten und zweiten Medium enthalten sind.

11. Die Vorrichtung (100) nach Anspruch 10, ferner umfassend
mindestens eine Elektrode (122), die in Kontakt mit dem ersten Volumen (18) steht,
eine zweite Messeinheit (124), die dazu eingerichtet ist, ein zweites elektrisches Signal zwischen mindestens einer Elektrode (122), die mit dem ersten Volumen (18) in Kontakt ist, und mindestens einer Elektrode (102), die mit dem zweiten Volumen (20) in Kontakt ist, zu messen, wobei die elektrische Steuereinheit (108) dazu eingerichtet ist, aus dem zweiten elektrischen Signal ein zweites Maß für die Permeabilität der Probe (16) zu bestimmen, wobei das zweite Maß zumindest teilweise eine Permeabilität der Probe (16) in Bezug auf Ionen, insbesondere anorganische Ionen, einer Elektrolytlösung charakterisiert, die mindestens eines von dem ersten und zweiten Medium bildet.

12. Vorrichtung nach Anspruch 10 oder 11, wobei der Probenhalter (14) zum Tragen einer Probe (16) ausgebildet ist, die zumindest teilweise aus biologischen Zellen besteht, und insbesondere durch eine Trägermembran (144) zum Tragen einer oder mehrerer Schichten biologischer Zellen gebildet ist, und/oder wobei die erste Messeinheit (106) dazu eingerichtet ist, eine Spannung zwischen mindestens zwei (102, 104) der mit dem zweiten Volumen (20) in Kontakt stehenden Elektroden anzulegen, insbesondere eine Wechselspannung, und den resultierenden Strom zwischen den Elektroden (102, 104) zu messen, und/oder wobei die zweite Messeinheit (124) dazu eingerichtet ist, eine Spannung zwischen mindestens einer mit dem ersten Volumen (18) in Kontakt stehenden Elektrode (122) und mindestens einer mit dem zweiten Volumen (20) in Kontakt stehenden Elektrode (102) anzulegen, insbesondere eine Wechselspannung, und den resultierenden Strom zwischen den Elektroden (102, 122) zu messen, und/oder wobei mindestens eine der ersten und der zweiten Messeinheit (106, 124) dazu eingerichtet ist, ein Maß für eine elektrische Impedanz zu bestimmen, und insbesondere ein Maß für eine elektrische Impedanz als Funktion einer Frequenz einer Wechselspannung oder eines Wechselstroms, die bzw. der zwischen mindestens zwei der mit der jeweiligen ersten oder zweiten Messeinheit (106, 124) verbundenen Elektroden (102, 104, 122) angelegt wird.

13. Vorrichtung nach einem der vorhergehenden Ansprüche 10-12, wobei eine Oberfläche von mindestens einer der Elektroden (102, 104, 122), die mit dem ersten (18) oder zweiten Volumen (20) in Kontakt steht,
parallel zu der zum ersten (18) oder zweiten Volumen (20) ausgerichteten Oberfläche der Probe (16) ist oder um weniger als 10°, vorzugsweise weniger als 5°, geneigt ist und
eine Fläche aufweist, die um weniger als 50 %, vorzugsweise weniger als 25 %, von der Fläche der zum ersten (18) oder zweiten Volumen (20) orientierten Oberfläche der Probe (16) abweicht, und/oder wobei mindestens zwei der mit demselben Volumen in Kontakt stehenden Elektroden ineinandergreifende Elektroden sind,
von denen jede einen Umfang hat, der mehr als das Zweifache, vorzugsweise mehr als das Fünffache, des Umfangs eines Kreises mit demselben Flächeninhalt wie eine Oberfläche der Elektrode beträgt.

14. Vorrichtung nach einem der Ansprüche 10 bis 13, wobei mindestens eine der mit dem zweiten Volumen (20) in Kontakt stehenden Elektroden (102, 104) eine kleine Elektrode ist, die eine Oberfläche aufweist, die weniger als 10%, vorzugsweise weniger als 5% der Oberfläche der zum zweiten Volumen (20) hin orientierten Probe (16) beträgt,
wobei die mindestens eine kleine Elektrode vorzugsweise eine einzelne kleine Elektrode (104) ist, die auf die Mitte der Oberfläche der zum zweiten Volumen (20) hin orientierten Probe (16) ausgerichtet ist, oder
wobei die mindestens eine kleine Elektrode vorzugsweise eine Vielzahl von kleinen Elektroden (104, 150-160) ist, die in einer Ebene verteilt sind, die parallel zur Oberfläche der zum zweiten Volumen (20) hin orientierten Probe (16) ist oder um weniger als 10°, vorzugsweise weniger als 5°, geneigt ist.

15. Vorrichtung nach einem der vorangehenden Ansprüche 10-14, wobei das erste Maß ein Permeabilitätskoeffizient der Probe (16) ist, und/oder wobei das zweite Maß ein elektrischer Widerstand der Probe (16) ist, und/oder wobei der Probenhalter (14) von der Vorrichtung abnehmbar ist.

## Revendications

1. Procédé pour la détermination de la perméabilité d'un échantillon (16) par l'observation d'un processus de diffusion par des moyens électrochimiques, ce procédé comprenant les étapes suivantes :

placement de l'échantillon (16) entre un premier volume (18) et un deuxième volume (20) pour former une barrière entre les deux volumes (18, 20),
la mise à disposition d'un premier milieu dans le premier volume (18) et d'un deuxième milieu dans le deuxième volume (20), dans lequel au moins un des premier et deuxième milieux contient au moins un type de molécules électrochimiquement actives et dans lequel les concentrations desdites molécules électrochimiquement actives dans lesdits premier et deuxième milieux sont différentes entre elles,
la mesure d'un premier signal électrique entre au moins deux électrodes (102, 104) en contact avec le deuxième volume (20) à différents moments tandis qu'on laisse se diffuser les molécules électrochimiquement actives à travers l'échantillon (16), ledit signal électrique dépendant de la concentration de molécules électrochimiquement actives à au moins une position dans le deuxième volume (20),
la détermination, à partir dudit premier signal électrique mesuré aux différents moments, d'une première mesure pour la perméabilité de l'échantillon (16), ladite première mesure caractérisant une perméabilité de l'échantillon (16) par rapport auxdites molécules électrochimiquement actives.

2. Procédé selon la revendication 1, dans lequel au moins un des premier et deuxième milieux est une solution d'électrolyte et dans lequel le procédé comprend en outre les étapes suivantes :

la mesure d'un deuxième signal électrique entre au moins une électrode (122) en contact avec le premier volume (18) et au moins une électrode (102) en contact avec le deuxième volume (20), ledit deuxième signal électrique dépendant de la conductivité électrique de l'échantillon (16) par rapport à un courant d'électrolyte et la détermination, à partir dudit deuxième signal électrique, d'une deuxième mesure pour la perméabilité de l'échantillon (16), ladite deuxième mesure caractérisant, au moins partiellement, une perméabilité de l'échantillon (16) par rapport à des ions, particulier des ions inorganiques, dudit électrolyte.

3. Procédé selon la revendication 2, dans lequel les premier et deuxième milieux ne contiennent initialement pas lesdites molécules électrochimiquement actives et lesdites molécules électrochimiquement actives sont ajoutées à au moins un des milieux après la mesure du deuxième signal électrique et avant la mesure du premier signal électrique.

4. Procédé selon l'une des revendications précédentes, dans lequel l'échantillon (16) est constitue, au moins partiellement, de cellules biologiques et/ou dans lequel ladite première mesure est un coefficient de perméabilité de l'échantillon (16) et/ou dans lequel ladite deuxième mesure est une résistance électrique de l'échantillon (16).

5. Procédé selon l'une des revendications précédentes, comprenant en outre les étapes suivantes :

la mesure d'au moins un troisième signal électrique entre au moins deux électrodes (102, 150) en contact avec le deuxième volume (20) à des moments différents tandis qu'on laisse les molécules électrochimiquement actives se diffuser à travers l'échantillon (16), ledit troisième signal électrique dépendant de la concentration des molécules électrochimiquement actives à au moins une position dans le deuxième volume (20), dans lequel au moins une des au moins deux électrodes (102, 150) est différente des au moins deux électrodes (102, 104) utilisées pour la mesure dudit premier signal électrique, la détermination, à partir dudit troisième signal électrique, d'une troisième mesure pour la perméabilité de l'échantillon (16), ladite troisième mesure caractérisant, au moins partiellement, une perméabilité de l'échantillon (16) par rapport auxdites molécules électrochimiquement actives.

6. Procédé selon la revendication 5, comprenant en outre :

la détermination, à partir dudit premier et dudit au moins troisième signal électrique, d'une information concernant la distribution spatiale d'une perméabilité de l'échantillon (16) par rapport auxdites molécules électrochimiquement actives, dans lequel le procédé comprend en outre de préférence l'obtention d'une mesure pour l'homogénéité de l'échantillon (16) à partir de ladite information concernant la distribution spatiale d'une perméabilité, la comparaison de ladite mesure d'homogénéité à des critères prédéfinis caractérisant l'homogénéité de l'échantillon (16) et la répétition des mesures avec un nouvel échantillon si lesdits critères ne sont pas respectés.

7. Procédé selon l'une des revendications précédentes, dans lequel au moins un des deux milieux est un fluide, plus particulièrement un liquide, ou un gel et/ou dans lequel le transfert desdites molécules électrochimiquement actives à travers l'échantillon (16) implique un transport paracellulaire.

8. Procédé selon l'une des revendications 2 à 7, dans lequel le transfert des ions dudit électrolyte à travers l'échantillon (16) implique un transport paracellulaire et/ou dans lequel lesdites molécules électrochimiquement actives sont plus grandes que les ions dudit électrolyte et/ou dans lequel le procédé comprend en outre la détermination, à partir dudit deuxième signal électrique, d'une mesure pour une capacité électrique de l'échantillon (16).

9. Procédé selon l'une des revendications précédentes, dans lequel au moins un des deux signaux électriques est mesure en appliquant une tension entre au moins deux desdites électrodes (102, 104, 122, 150), plus particulièrement une tension alternative, et en mesurant le courant qui en résulte entre les électrodes et/ou dans lequel le procédé comprend en outre la détermination, à partir d'au moins un desdits premier et deuxième signaux électriques, d'une mesure pour une impédance électrique, et plus particulièrement d'une mesure pour une impédance électrique en fonction d'une fréquence d'une tension alternative ou d'un courant alternatif appliqué entre les au moins deux électrodes utilisées pour la mesure du premier ou deuxième signal électrique respectif.

**10.** Dispositif (100) pour la détermination de la perméabilité d'un échantillon (16) par l'observation d'un processus de diffusion par des moyens électrochimiques, ce dispositif comprenant :

un premier volume (18) pour contenir un premier milieu,

un deuxième volume (20) pour contenir un deuxième milieu, le deuxième volume (20) étant relié au premier volume (18),

un support d'échantillon (14) pour monter l'échantillon (16) de façon à ce que l'échantillon (16) forme une barrière entre les deux volumes (18, 20),

au moins deux électrodes (102, 104) en contact avec le deuxième volume (20),

une première unité de mesure (106) conçue pour mesure un premier signal électrique entre au moins deux des électrodes (102, 104) en contact avec le deuxième volume (20) à différents moments,

une unité de contrôle électrique (108) conçue pour déterminer, à partir dudit premier signal électrique mesuré aux différents moments, une première mesure pour la perméabilité de l'échantillon (16), ladite première mesure caractérisant une perméabilité de l'échantillon (16) par rapport aux molécules électrochimiquement actives contenues dans au moins un desdits premier et deuxième milieux.

**11.** Dispositif (100) selon la revendication 10, comprenant en outre :

au moins une électrode (122) en contact avec le premier volume (18),

une deuxième unité de mesure (124) conçue pour mesure un deuxième signal électrique entre au moins une électrode (122) en contact avec le premier volume (18) et au moins une électrode (102) en contact avec le deuxième volume (20),

dans lequel l'unité de contrôle électrique (108) est conçue pour déterminer, à partir dudit deuxième signal électrique, une deuxième mesure pour la perméabilité de l'échantillon (16), ladite deuxième mesure caractérisant, au moins partiellement, une perméabilité de l'échantillon (16) par rapport à des ions, plus particulièrement des ions inorganiques, d'une solution d'électrolyte formant au moins un desdits premier et deuxième milieux.

**12.** Dispositif selon la revendication 10 ou 11, dans lequel le support d'échantillon (14) est conçu pour supporter un échantillon (16) constitué, au moins partiellement, de cellules biologiques et plus particulièrement constitué d'une membrane de support (144) pour supporter une ou plusieurs couches de cellules biologiques et/ou

dans lequel la première unité de mesure (106) est conçue pour appliquer une tension entre au moins deux desdites électrodes (102, 104) en contact avec le deuxième volume (20), plus particulièrement une tension alternative, et pour mesurer le courant qui en résulte entre les électrodes (102, 104) et/ou

dans lequel la deuxième unité de mesure (124) est conçue pour appliquer une tension entre au moins une électrode (122) en contact avec le premier volume (18) et au moins une électrode (102) en contact avec le deuxième volume (20), plus particulièrement une tension alternative, et pour mesurer le courant qui en résulte entre les électrodes (102, 122) et/ou

dans lequel au moins une desdites premier et deuxième unités de mesure (106, 124) est conçue pour déterminer une mesure pour une impédance électrique et plus particulièrement une mesure pour une impédance électrique en fonction d'une fréquence d'une tension alternative ou d'un courant alternatif appliqué entre au moins deux des électrodes (102, 104, 122) reliées respectivement aux première et deuxième unités de mesure (106, 124).

**13.** Dispositif selon l'une des revendications précédentes 10 - 12, dans lequel une surface d'au moins une des électrodes (102, 104, 122) en contact avec le premier volume (18) ou le deuxième volume (20) est parallèle à la surface de l'échantillon (16) orientée en direction du premier volume (18) ou du deuxième volume (20) ou inclinée de moins de 10°, de préférence de moins de 5° et

présente une superficie différente de moins de 50 %, de préférence de moins de 25 % de la superficie de l'échantillon (16) orientée en direction du premier volume (18) ou du deuxième volume (20) et/ou

dans lequel au moins deux des électrodes en contact avec le même volume sont des électrodes interdigitées,

chacune d'elles présente un périmètre qui est plus de deux fois, de préférence plus de cinq fois plus grand que la circonférence d'un cercle avec la même superficie qu'une surface de l'électrode.

**14.** Dispositif selon l'une des revendications 10 - 13, dans lequel au moins une des électrodes (102, 104) en contact avec le deuxième volume (20) est une petite électrode qui présente une superficie qui représente moins de 10 %, de préférence moins de 5 % de la superficie de l'échantillon (16) orientée en direction du deuxième volume (20),

dans lequel au moins une petite électrode est de préférence une petite électrode simple (104) qui est alignée avec le centre de la surface de l'échantillon (16) orientée en direction du deuxième volume (20) ou

dans lequel ladite au moins une petite électrode est de préférence une pluralité de petites électrodes (104, 150 -160) qui sont réparties dans un plan qui est parallèle à la surface de l'échantillon (16) orientée en direction du deuxième volume (20) ou inclinée de moins de 10°, de préférence de moins de 5°.

15. Dispositif selon l'une des revendications 10 - 14, dans lequel ladite première mesure est un coefficient de perméabilité de l'échantillon (16) et/ou
dans lequel ladite deuxième mesure est une résistance électrique de l'échantillon (16) et/ou
dans lequel le support d'échantillon (14) peut être retiré du dispositif.

Fig. 1a

Fig. 1b

# Fig. 2a

# Fig. 2b

Fig. 2c

place sample
between volumes     112

insert media
into volumes     114

measure first
electrical signal
during diffusion     116

determine first
permeability measure     118

# Fig. 3a

Fig. 3b

```
┌─────────────────────┐
│    place sample     │ ─── 130
│   between volumes   │
└─────────────────────┘
           │
           ▼
┌─────────────────────┐
│    insert media     │ ─── 132
│    into volumes     │
└─────────────────────┘
           │
           ▼
┌─────────────────────┐
│    measure first    │ ─── 134
│  electrical signal  │
│   during diffusion  │
└─────────────────────┘
           │
           ▼
┌─────────────────────┐
│   determine first   │ ─── 136
│ permeability measure│
└─────────────────────┘
           │
           ▼
┌─────────────────────┐
│   measure second    │ ─── 138
│  electrical signal  │
└─────────────────────┘
           │
           ▼
┌─────────────────────┐
│  determine second   │ ─── 140
│ permeability measure │
└─────────────────────┘
```

Fig. 4

Fig. 5a

Fig. 5b

Fig. 5c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **D. GÜNZE et al.** *Curr. Top. Membr.,* 2010, vol. 65, 39-78
- **J. WEGENER et al.** *Cell Tissue Res.,* 2014, vol. 335, 485-514
- **S. SANDERS et al.** *Epithelial Cell Biol.,* 1995, vol. 4, 25-34
- **H. GHANDEHARI et al.** *J. Pharm. Exp. Ther.,* 1997, vol. 280, 747-753
- **J. WEGENER et al.** *J. Biochem. Biophys. Methods,* 1996, vol. 32, 151
- **K. HAJEK ; J. WEGENER.** *Experimental Cell Research,* 2017, vol. 351, 121
- **J. WEGENER et al.** *Brain Research,* 2000, vol. 853, 115
- **J. L. WOOD et al.** *J. Exp. Biol.,* 1978, vol. 77, 123-140
- **K, MORIMOTO et al.** *Pharm. Res.,* 1993, vol. 10, 1668-1674
- **H. GHANDEHARI et al.** *J. Pharm. Exp. Ther,* 1997, vol. 280, 747-753
- **K. MORIMOTO et al.** *Pharm. Res.,* 1993, vol. 10, 1668-1674
- **D. GÜNZEL et al.** *Curr. Top. Membr.,* 2010, vol. 65, 39-78